# EUROPEAN PATENT SPECIFICATION

(11) **EP 2 407 155 B1**
(45) Date of publication and mention of the grant of the patent: **26.12.2012**
(21) Application number: 11173860.5
(22) Date of filing: 13.07.2011
(51) Int. Cl.: A61K 9/10, A61K 9/20, A61K 9/00, A61K 9/48, A61K 47/10, A61K 9/08

(54) **Formulations of inecalcitol**
Formulierungen des Inecalcitols
Formulations de l'inécalcitol

(30) Priority: 15.07.2010 US 836974; 15.07.2010 EP 10305783
(43) Date of publication of application: 18.01.2012
(73) Proprietor: Hybrigenics S.A., 75014 Paris (FR)
(72) Inventor: Delansorne, Rémi, 75006 PARIS (FR)
(74) Representative: Jacobson, Claude

(56) References cited:
- WO-A1-2006/042383
- JP-A- 2010 111 593
- US-A1- 2007 093 458
- STRICKLEY R G: "SOLUBILIZING EXCIPIENTS IN ORAL AND INJECTABLE FORMULATIONS", PHARMACEUTICAL RESEARCH, KLUWER ACADEMIC PUBLISHERS, NEW YORK, NY, US, vol. 21, no. 2, 1 February 2004 (2004-02-01), pages 201-230, XP009035738, ISSN: 0724-8741, DOI: DOI:10.1023/B:PHAM.0000016235.32639.23
- "Promising interim results from Hybrigenics' Inecalcitol Phase II in combination with Taxotere(R) for hormone refractory prostate cancer", INTERNET CITATION, 19 April 2009 (2009-04-19), pages 1-3, XP002592440, Retrieved from the Internet: URL:http://www.medicalnewstoday.com/articl es/146552.php [retrieved on 2010-07-08]
- MANUS ET AL: "Inecalcitol, traitement du cancer de la prostate", REVUE FRANCOPHONE DES LABORATOIRES, ELSEVIER, AMSTERDAM, NL, vol. 2008, no. 405, 1 October 2008 (2008-10-01), page 12, XP025686090, ISSN: 1773-035X, DOI: DOI:10.1016/S1773-035X(08)74264-3 [retrieved on 2008-10-01]

## Description

The present invention concerns improved formulations of 14-epi-analogues of vitamin D.

Vitamin D belongs to the group of fat-soluble vitamins, together with vitamins A, E and K. The two main natural forms of vitamin D: vitamin D₂ (also named ergocalciferol) and vitamine D₃ (also named cholecalciferol) are soluble in lipids and insoluble in water, like all their chemical derivatives.

Vitamin D, its metabolites and analogues have potent effects on calcium and phosphate metabolism and can therefore be used for prevention and therapy of vitamin D deficiency, such as rickets and other disorders of plasma and bone mineral homeostasis such as osteoporosis and osteomalacia. Moreover, vitamin D receptors and vitamin D activity have also been documented in numerous other tissues and cells, where they are also known to be involved in cell proliferation and differentiation. Vitamin D also affects the immune system as vitamin D receptors are expressed in several white blood cells including monocytes, macrophages and T and B lymphocytes.

Pharmaceutical formulations of vitamin D compounds as active pharmaceutical ingredients show a large variety: ergocalciferol is available in solution in ethanol for oral administration or in arachis oil for injection since 1940 ; cholecalciferol is marketed as a solution in medium-chain triglycerides (MCT) both for oral and injectable administrations since 1964 ; in 1974, a droplet formulation of calcifediol (25-hydroxy-cholecalciferol) in solution in propylene glycol was introduced; in 1979, alfacalcidol (1-hydroxy-cholecalciferol) was approved for oral administration as soft gelatin capsules filled with solution in sesame oil; in 1982, calcitriol (1,25-dihydroxy-cholecalciferol) was launched in soft gelatin capsules filled with solution in MCT; in 1989, a new formulation of cholecalciferol was made available as drinking solution in a complex mixture of polyoxyethylenated glycerides, propylene glycol, glycerol, Cremophor EL and phosphate buffer ; in the same year, a new formulation of ergocalciferol was also marketed in a complex mixture of polyoxyethyletaned and glycosylated glycerides ; in 2000, a new simple solution of cholecalciferol in olive oil was introduced as a drinking solution ; the two latest innovative vitamin D compounds to have been approved for oral pharmaceutical use, doxercalciferol in 1999 and paricalcitol in 2005, are both formulated as solution in MCT in soft gelatin capsules; finally, elocalcitol has recently been developed up to phase IIb clinical trials in soft gelatin capsules containing MCT (US7,332,482).

The absorption of cholecalciferol has been compared when given to human volunteers in solution in identical soft gelatin capsules containing either arachis oil or MCT (Miglyol 812®) : in the fasting state, bioavailability of cholecalciferol was about twice higher from arachis oil capsules than from MCT capsules (Holumberg et al., 1990, Biopharm. Drug Disp., 11, 807-8015). When given with a standard meal, no difference was observed.

The bioavailability of seocalcitol, a cholecalciferol derivative, was studied in rats in solution either in propylene glycol as the reference formulation, or in two types of lipids: MCT or long chain triglycerides (LCT) (Grove et al., 2005, J. Pharm. Sci., 8, 1830-1838). A two-fold increase in bioavailability of seocalcitol was demonstrated in both lipid formulations over the reference solution in propylene-glycol; no difference was observed between the LCT- or MCT-based formulations.

The optimization of the oral delivery of lipophilic compounds in lipid-based formulations has been reviewed (Porter et al., Nature Rev. Drug Discovery, 2007, 6, 231-248). Differences between MCT and LCT on different mechanisms involved in the efficiency of lipophilic drug bioavailability were listed such as drug solubilisation, reduction in gastro-intestinal motility, lymphatic versus hepatic portal transport, secretion of biliary salts and of endogenous phospholipids, or the dynamics of lamellar versus cubic liquid crystal phases in the course of lipid droplet digestion into vesicles, mixed micelles and simple micelles.

The complexity of lipid drug absorption has sometimes led to complex formulations incorporating several components playing several different roles such as solvent, surfactant, co-solvent, co-surfactant and the like. Such a complex system has been tested with seocalcitol without any improvement in bioavailability as compared with simple solutions in MCT or LCT (Grove et al., 2006, Eur. J. Pharma. Sci., 28, 233-242).

In summary, *"the choice of specific formulation components to provide optimal pharmaceutical and biopharmaceutical properties is drug specific and will depend on drug dose and the physicochemical properties of the compound concerned* " (Porter et al., *ibid,* 2007, page 239, box 3). This explains the variety of oral formulations for the variety of vitamin D compounds, although soft gelatin capsules appear to be the most popular galenic form and MCT the most frequent excipient.

On the other hand, these so-called non-calcemic effects of vitamin D lead to consider the possible use of vitamin D derivatives for various therapeutic applications such as disorders of the immune system, hormone secretion, cell differentiation or cell proliferation. In particular, such compounds may be useful in the therapy of disorders characterized by increased cell proliferation, such as psoriasis and/or cancer. In particular, 1,25(OH)₂-vitamin D₃, the active metabolite of vitamin D₃ named calcitriol, is known to inhibit the proliferation of many cancer cells lines of various origins *in vitro* and to slow the progression of various tumor xenografts *in vivo.*

A large number of analogues of calcitriol displaying a clear dissociation between anti-proliferative and calcemic effects have been reported. In particular, EP 0 707 566 B1 discloses a number of calcitriol analogues such as 14-epi-analogues. Among these 14-epi-analogues of calcitriol is inecalcitol of formula:

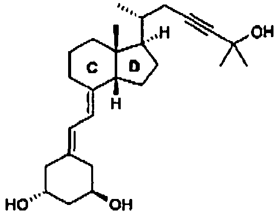

Inecalcitol is the international non-proprietary name for 19-nor-9,10-seco-14βH-cholesta-5(Z),7(E)-dien-23-ino-1α,3β,25-triol-23-yne (C₂₈H₄₀O₃).

Inecalcitol is a synthetic derivative of calcitriol, the natural active metabolite of vitamin D₃. Eelen et al. (Molecular Pharmacology 67, 1566-1573, 2005) and Verlinden et al. (Journal of Bone and Mineral Research, volume 16(4), 625-638, 2001) showed the enhanced antiproliferative and markedly lower calcemic effects of inecalcitol compared with calcitriol.

Inecalcitol is an original vitamin D analogue different from all other vitamin D derivatives by the *cis* conformation of the junction between the C and D rings of the molecule, rather than the *trans* configuration in the natural vitamin D compounds (Verlinden et al., 2000, Cancer Res., 60. 2673-2679). This modification, also called 14-epimerization, has been described for the first time by Maynard et al. (1994, J. Med. Chem., 37, 2387-2393) in the vitamin D series, and only very seldom applied to synthesize new 14-epi-vitamin D analogues (W0 2004 080922 and related patent documents; US 5,936,105; W0 95/01960 and related patent documents; Sawada et al., 2009, Bioorg. Med. Chem., 19, 5397-5400; Sawada et al., 2009, Chem. Pharm. Bull, 57, 1431-1433; Kittaka et al., 2009, Anticancer Res., 9, 3563-3569. Sawada et al.; 2010, J. Steroid Biochem. Mol Biol.*,* doi;10.1016/j.jsbmb.2010.02.035). Inecalcitol is the most advanced 14-epi vitamin D derivative in terms of clinical development: it has been tested both in healthy volunteers and in human patients by oral administration in the classical formulation as a solution in MCT in soft gelatin capsules, like elocalcitol, paricalcitol, doxercalciferol, calcitriol and the first formulation of cholecalciferol.

A major feature of inecalcitol profile is its very low calcemic activity, especially in monkeys and human subjects, allowing the oral administration of very high doses. In humans, the maximal tolerated (MDT) dose is 4 mg per day, much higher than the MTD of any other vitamin D analogue previously determined: 10 µg per day for seocalcitol, 45 µg once a week for calcitriol and 150 µg per day for elocalcitol.

Thus, inecalcitol, as a representative of a unique physico-chemical class of 14-epiderivatives of vitamin D, also differs for the range of dose at which it can be administrated. Both reasons may explain the unpredictable results obtained with high dose formulations of inecalcitol orally administrated to monkeys as test animals, more representative of human tolerance than rats.

In view of the unique structure and doses, the usual formulations of vitamin D derivatives may not be optimized for the 14-epi-analogues. It is thus desirable to provide improved formulations of inecalcitol.

It is an object of the present invention to provide new oral formulations of inecalcitol, showing an improved absorption profile over the Medium Chain Triglycerides (MCT) formulation of said 14-epi-analogues of vitamin D, in particular in terms of the peak concentration (Cmax).

Preferably, the formulations of the invention exhibit at least two-fold increase of the Cmax peak of the MCT formulation in mammals, such as in human and/or monkeys.

The formulations of the invention may also exhibit additionally or alternatively an improved overall bioavailability (AUC₀₋₂₄ₕ) over the Medium Chain Triglycerides formulation of said 14-epi-analogues of vitamin D. Preferably, the formulations of the invention lead to a higher AUC₀₋₂₄ₕ than the Medium Chain Triglycerides formulation of said 14-epi-analogues of vitamin D in mammals, such as in human and/or monkeys.

According to a first object, the present invention concerns an oral formulation of the 14-epi-analogue of vitamin D characterized by an improved absorption profile over the Medium Chain Triglycerides (MCT) formulation of said 14-epi-analogues of vitamin D.

According to a second object, the present invention also concerns an oral formulation of the 14-epi-analogue of vitamin D for use for improving the absorption of said 14-epi-analogue over the formulation in medium chain triglycerides.

The improved absorption profile is such that the oral formulations of the invention have a higher peak concentration (Cmax) and may additionally exhibit a higher overall absorption (AUC₀₋₂₄ₕ) than the corresponding formulation in MCT of said 14-epi-analogue of vitamin D.

As used herein, the term "14-epi-analogue of vitamin D" refers to inecalcitol.

In the oral formulations of the invention, the concentration of said 14-epi-analogue in said formulation is comprised between 0.001 and 50 % (weight).

The oral formulations of the invention are in the form of a soft gelatin capsule comprising :
- inecalcitol,
- at least one Long Chain Triglyceride (LCT) chosen among olive oil, sunflower oil and their mixtures
and one or more optional pharmaceutically acceptable excipient(s).

Generally, in the oral formulation, the concentration (weight) of LCT is comprised between 1% and qsp.

It has also been surprisingly discovered that formulations of inecalcitol with Long Chain Triglycerides (LCT) lead to an improved absorption over the corresponding MCT formulations of said 14-epi-analogues. This result is unexpected as this improved activity is not obtained with non esterified corresponding long-chain fatty acid like oleic acid.

The terms "Long Chain Triglyceride" refer to long chains comprising more than 14 carbon atoms fatty esters of glycerol. Preferably they comprise 15 to 24 carbon atoms. According to the invention, Long Chain Triglyceride are olive, or sunflower oils, and their mixtures.

By contrast, MCT refers to Medium-chain triglycerides (MCTs), comprising 6 to 14 carbons fatty acid esters of glycerol: caproic acid, caprylic acid, capric acid, lauric acid, myristic acidor myristoleic acid, and their derivatives MCT are found in vegetable oils such as coconut oil or palm kernel oil.

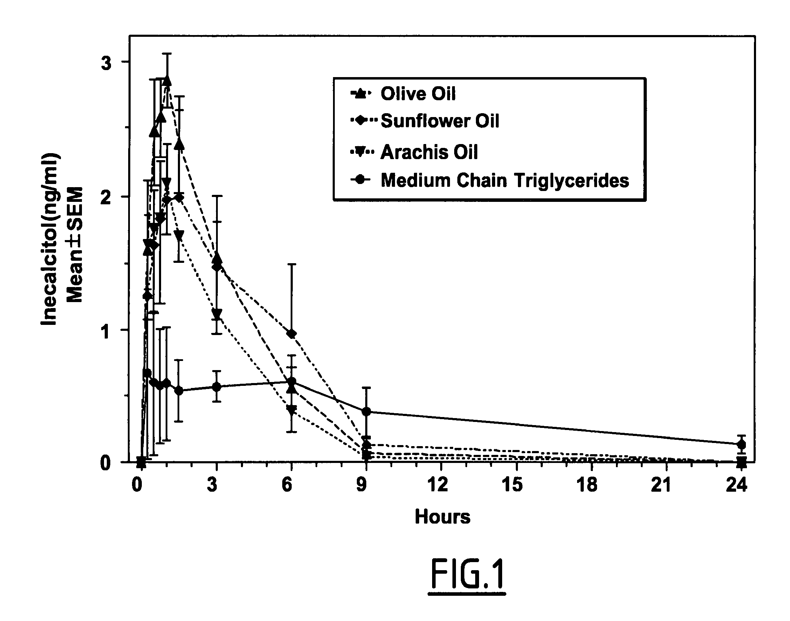

Specifically, inecalcitol was better absorbed from LCT (arachis oil, sunflower oil, olive oil) than from MCT as apparent from the results illustrated in Figure 1.

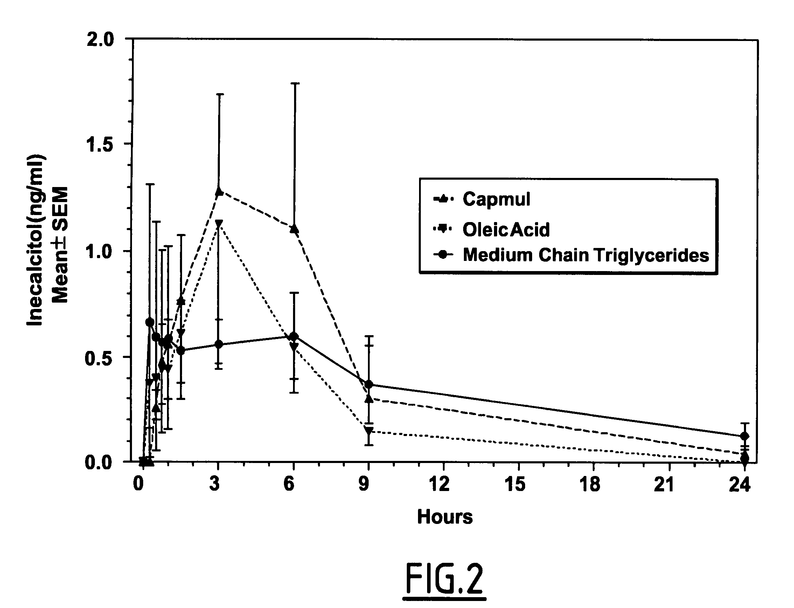

Long-chain fatty acid like oleic acid, the majority fatty acid esterified in the olive oil triglycerides, changed the course of inecalcitol absorption by delaying and slightly increasing its peak concentration, but reduced its overall bioavailability, as illustrated in Figure 2.

Soft gelatin capsules are made from gelatin blends, optionally blended with plasticizers such as glycerol, glycerin, sorbitol and sorbitol derivatives. Soft gelatin capsules can be filled with various solutions or emulsions of pharmaceutically active ingredient in LCT alone or mixed with other excipients like surfactants such as polyethyleneglycerides, polysorbates, hydrogenated castor oil and the like, emulsifyers such as phospholipids, semi-synthetic mono-or di-glycerides, propylene glycol, esters of propylene glycol, esters of fatty acids, cholesterol derivatives, lecithins, and the like, or antioxydants such as butylhydroxyanisole, butylhydroxytoluene, ascorbic acid, citric acid, tocopherols or alpha-tocopherol, and the like.

"Qsp" is used herein to indicate that the ingredient is present in the oral formulation in an amount necessary and/or sufficient to achieve the desired final weight of said formulation. It is thus used to refer to the complementary part or balance quantity of said ingredient.

The oral formulations of the invention may be in the form of a unit dose. Such unit doses generally comprise from 0.5 to 20 mg of said 14-epi-analogue.

The oral formulations are suitable for use in the prevention and/or treatment of rickets, osteoporosis, osteomalacia, psoriasis, autoimmune diseases such as multiple sclerosis or type I diabetes, hyperparathyroidism, benign prostate hyperplasia, any type of cancer or any vitamin D associated disease.

According to a further object, the present invention concerns a method for treating and/or preventing rickets, osteoporosis, osteomalacia, psoriasis, autoimmune diseases such as multiple sclerosis or type I diabetes, hyperparathyroidism, benign prostate hyperplasia, any type of cancer or any vitamin D associated disease, in particular cancer, comprising administering a formulation of the invention to a human or animal patient in need thereof.

The formulations of the invention may provide any therapeutically effective amount of said 14-epi-analogue. The administration dose of said 14-epi-analogue is preferably comprised between 1.5 mg and 20 mg.

The method of the invention advantageously does not induce increased calcemia in the treated patient.

The method of the invention may comprise the administration of said doses of the 14-epi-analogue, at a frequency comprised between every three days up to three times a day, such as every three days, every other day (qod), once-a-day (qd), twice-a-day (bid) or three times a day (tid). Preferably, the administration may take place every other day, once a day or twice-a-day.

As used herein, a "therapeutically effective amount" refers to an amount of a 14-epi-analogue which is effective in preventing, reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment. The identification of those subjects who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

The amount of the 14-epi-analogue, which is required to achieve the desired biological effect, will vary depending upon a number of factors, including the chemical characteristics (e.g. hydrophobicity) of the compounds employed, the potency of the compounds, the type of disease, the species to which the patient belongs, the diseased state of the patient, the route of administration, the bioavailability of the compound by the chosen route, all factors which dictate the required dose amounts, delivery and regimen to be administered.

In the context of the invention, the term "treating" or "treatment", as used herein, means reversing, alleviating, inhibiting the progress of, or preventing the disorder or condition to which such term applies, or one or more symptoms of such disorder or condition.

According to the invention, the terms "patient" or "patient in need thereof" are intended for an animal or a human being affected or likely to be affected with a pathological condition as discussed above. Preferably, the patient is human.

As used herein, "pharmaceutically acceptable excipient" includes any carriers, diluents, adjuvants or vehicles, such as preserving or antioxidant agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well-known in the art. Except insofar as any conventional media or agent is incompatible with the active ingredient, its use in the therapeutic compositions is contemplated. Supplementary active ingredients can also be incorporated into the compositions as suitable therapeutic combinations.

The formulations of the invention may be prepared by admixture of their ingredients.

The formulations may conveniently be administered in unit dosage form and may be prepared by any of the methods well-known in the pharmaceutical art, for example, as described in Remington: The Science and Practice of Pharmacy, 20th ed.; Gennaro, A. R., Ed.; Lippincott Williams & Wilkins: Philadelphia, PA, 2000.

### Figures

Figure 1 shows the time-dependent concentration of inecalcitol following its oral administration in LCT (olive oil, sunflower oil and arachis oil) or in MCT in monkeys.
Figure 2 shows the time-dependent concentration of inecalcitol following its oral administration with capmul, oleic acid or MCT in monkeys, for comparative purposes.
Figure 3 shows the time-dependent concentration of inecalcitol following its oral administration with propylene glycol in monkeys.
Figure 4 shows the time-dependent concentration of inecalcitol following its oral administration with CMC in monkeys.

The following examples are given for illustrative and non-limiting purpose.

### Example 1

Inecalcitol was dissolved in arachis oil, olive oil, sunflower oil (0.4 mg/ml, i.e. 0.044% w/w), propylene glycol (0.4 mg/ml, i.e. 0.039% w/w), oleic acid (0.4 mg/ml, i.e. 0.045% w/w), MCT (0.4 mg/ml, i.e. 0.042% w/w), or Capmul PG8® (0.4 mg/ml, i.e. 0.044% w/w), or dispersed as a fine milky suspension in pharmaceutical grade distilled water containing 0.5% (w/v) carboxymethylcellulose, corresponding to a 3.33% w/w concentration in the tablet formulation (5 mg CMC and 2 mg Inecalcitol in each tablet weighing 150 mg without coating). Male cynomolgus monkey received a single oral administration of a high dose of inecalcitol (2 mg/kg) in the same volume (5 ml/kg) for each excipient. Blood samples were drawn at the time of administration (To) and 15, 30, 45, 60, 90 minutes, 3, 6, 9 and 24 hours later. Plasma aliquots were prepared on heparinized tubes and unchanged inecalcitol was specifically assayed by HPLC/MS/MS method. Mean pharmacokinetic profiles (± SEM) are plotted in figures 1 to 4; the mean maximum concentrations (Cmax) and area under the curve over 24 hours after administration (AUC₀₋₂₄ₕ) were expressed in relation to the mean value observed with the reference solution of inecalcitol in MCT (Table 1); the median time (Tmax) at which Cmax was observed in the different animals receiving the same treatment was determined (Table 1).

**Table 1**

| Excipient | Cmax (-fold) | Tmax (h) | AUC_{0 -24h} (%) | N |
|---|---|---|---|---|
| MCT | 1 | 0,25 | 100 | 6 |
| Oleic acid | 1.7 | 3 | 67 % | 4 |
| Capmul PG8 | 1.9 | 3 | 121 % | 3 |
| Arachis oil | 3.2 | 1 | 91 % | 4 |
| Sunflower oil | 3.0 | 1.5 | 132 % | 4 |
| Olive oil | 4.3 | 1 | 127 % | 4 |
| CMC-water | 11.6 | 0.5 | 166 % | 9 |
| Propylene glycol | 16.5 | 0.75 | 193 % | 3 |

| | | | | |
|---|---|---|---|---|
| N: Total number of monkeys per formulation | | | | |

It appears that inecalcitol was better absorbed from LCT (arachis oil, sunflower oil, olive oil) than from MCT (figure n°1; Table 1). Long-chain fatty acid like oleic acid, the majority fatty acid esterified in the olive oil triglycerides, changed the course of inecalcitol absorption by delaying and slightly increasing its peak concentration (figure 2; Table 1).

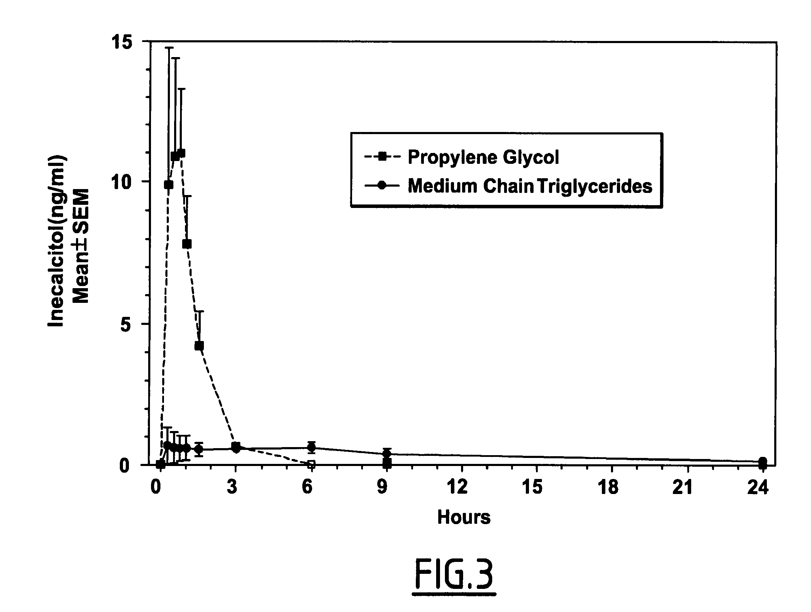

It also appears that solution of inecalcitol in propylene glycol was even more readily absorbed to give rise to peak values more than 15-fold higher than from in MCT. The resulting total bioavailability was nearly doubled (figure 3; Table 1). However, the monoester of propylene glycol with caprylic acid (CapmuIPG8®) increased inecalcitol peak plasma concentration only by a factor of 2, for an increase in bioavailability limited to about 20% (figure 2; Table 1).

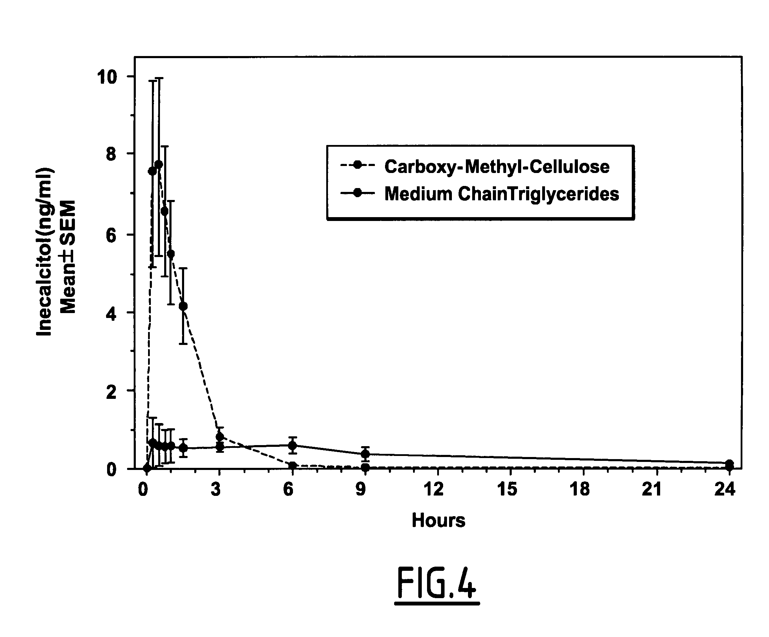

Even more striking, when inecalcitol was administrated to monkeys in suspension in a water solution of the hydrophilic linking/disintegrating excipient carboxymethylcellulose (CMC), the absorption was better than from any solution of inecalcitol in any lipid-based excipient. Peak concentration was almost 12-fold higher with suspensions in CMC solutions than with solution in MCT. The overall bioavailability was increased by 66% (figure 4; Table 1).

### Example 2: Composition of 2 mg Inecalcitol tablets

| Component | Composition centesimal (%) | Unit composition (mg) |
|---|---|---|
| Inecalcitol | 1.333 | 2,000 |
| Lactose monohydrate | 66.967 | 100.451 |
| Cellulose microcristalline ph102 | 11.000 | 16.500 |
| Carboxymethyl cellulose sodique | 3.333 | 5.000 |
| Crospovidone | 15.000 | 22.500 |
| Anhydrous collidal silicon dioxide | 0.500 | 0.750 |
| Dry vitamin E 50% | 1.667 | 2.501 |
| Magnesium stearate | 0.200 | 0.300 |
| **Total raw tablet** | **100.000** | **150.002** |
| Opadry II white 85F18422 | | 3.000 |
| **Total coated tablet** | | **153.002** |

## Claims

1. An oral formulation wherein said formulation is a soft gelatin capsule comprising :
- inecalcitol,
- at least one Long Chain Triglyceride (LCT) chosen among olive oil, sunflower oil and their mixtures , and
- one or more optional pharmaceutically acceptable excipient(s).

2. The oral formulation according to claim 1, wherein the concentration (weight) of LCT is comprised between 1 % and qsp.

3. The oral formulation according to anyone of the preceding claims, wherein the concentration of inecalcitol is comprised between 0.001 and 50% (weight).

4. The oral formulation according to anyone of the preceding claims in a unit dose.

5. The oral formulation according to anyone of the preceding claims, comprising from 0.5 to 20 mg of inecalcitol.

6. The oral formulation according to anyone of claims 1 to 5 **characterized by** an improved absorption profile over the Medium Chain Triglycerides (MCT) formulation of inecalcitol.

7. The oral formulation according to anyone of preceding claims in the form of a soft gelatin capsule comprising a solution or an emulsion of pharmaceutically active ingredient in LCT alone or mixed with other excipients, emulsifiers or antioxidants, and the like.

8. The oral formulation according to anyone of the preceding claims for use in the prevention and/or treatment of rickets, osteoporosis, osteomalacia, psoriasis, autoimmune diseases such as multiple sclerosis or type I diabetes, hyperparathyroidism, benign prostate hyperplasia, any type of cancer or any vitamin D associated disease.

9. The oral formulation according to anyone of claims 1 to 8 for use for improving the absorption of inecalcitol over the formulation in medium chain triglyceride.

## Patentansprüche

1. Orales Präparat, wobei das genannte Präparat eine Weichgelatinekapsel ist, die enthält:
Inecalcitol,
- mindestens ein langkettiges Triglycerid (LCT), ausgewählt unter Olivenöl. Sonnenblumenöl und ihren Gemischen, und
- optional ein oder mehrere pharmazeutisch akzeptierbare Vehikel

2. Orales Präparat nach Patentanspruch 1, in dem die Konzentration (Gewicht) an LCT zwischen 1% und q.s.p. liegt.

3. Orales Präparat nach irgendeinem der vorangehenden Patentansprüche, in dem die Konzentration von inecalcitol zwischen 0,001 und 50% (Gewicht) beträgt.

4. Orale Präparat nach irgendeinem der vorangehenden Patentansprüche als Einzeidosis,

5. Orales Präparat nach irgendeinem der vorangehenden Patentansprüche, 0,5 bis 20 mg Inecalcitol enthaltend.

6. Orales Präparat nach irgendeinem der Patentansprüche 1 bis 5, **gekennzeichnet durch** ein verbessertes Absorptionsprofil gegenüber Inecalcitot-Präparaten mit mittelkettigen Triglyceriden (MCT).

7. Orales Präparat nach irgendeinem der vorangehenden Patentansprüche in Form einer Weichgelatinekapsel, die eine Lösung oder Emulsion pharmazeutisch wirksamer Bestandteile in LCT, alleine oder gemischt mit anderen Vehikeln, Emulgatoren oder Antioxidantien und Ähnlichem enthält.

8. Orales Präparat nach irgendeinem der vorangehenden Patentansprüche zum Gebrauch in der Vorbeugung und/oder Behandlung von Rachitis, Osteoporose, Osteomalazie, Psoriasis, Autoimmunkrankheiten wie multiple Sklerose oder Diabetes Typ 1, Hyperparathyreoidismus, gutartige Prostatahyperplasie, einer beliebigen Krebsart oder jeglicher Krankheit, die mit Vitamin D verbunden ist.

9. Orales Präparat nach irgendeinem der Patentansprüche 1 bis 8 zum Gebrauch zur Verbesserung der Inecalcitol-Absorption gegenüber Präparaten mit mittelkettigen Triglyceriden

## Revendications

1. Préparation orale, dans laquelle ladite préparation est une capsule de gélatine molle contenant :
- de l'inécalcitol,
- au moins un triglycéride à chaîne longue (TCL) choisi parmi l'huile d'olive, l'huile de tournesol et leurs mélanges, et
- un ou plusieurs excipients pharmaceutiquement acceptables facultatifs.

2. Préparation orale selon la revendication 1, dans laquelle la concentration (en poids) en TCL est comprise entre 1 % et qsp.

3. Préparation orale selon l'une quelconque des revendications précédentes, dans laquelle la concentration en inécalcitol est comprise entre 0,001 % à 50% (en poids).

4. Préparation orale selon l'une quelconque des revendications précédentes, dans une dose unitaire.

5. Préparation orale selon l'une quelconque des revendications précédentes, comprenant 0,5 mg à 20 mg d'inécalcitol.

6. Préparation orale selon l'une quelconque des revendications 1 à 5, **caractérisée par** un profil d'absorption améliorée par rapport à la préparation d'inécalcitol contenant des triglycérides à chaîne moyenne (TCM).

7. Préparation orale selon l'une quelconque des revendications précédentes, sous la forme d'une capsule de gélatine molle contenant une solution ou une émulsion d'un principe pharmaceutiquement actif dans des TCL, seul ou en mélange avec d'autres excipients, émulsionnants ou antioxydants, et équivalents,

8. Préparation orale selon l'une quelconque des revendications précédentes, pour une utilisation dans la prévention et/ou le traitement du rachitisme, de l'ostéoporose, de l'ostéomalacie, du psoriasis, des maladies auto-immunes telles que la sclérose en plaques ou le diabète de type 1, de l'hyperparathyroïdisme, de l'hyperplasie bénigne de la prostate, de n'importe quel type de cancer ou de n'importe quelle maladie associée à la vitamine D.

9. Préparation orale selon l'une quelconque des revendications 1 à 8, pour une utilisation pour améliorer l'absorption de l'inécalcitol par rapport à la préparation contenant des triglycérides à chaîne moyenne.
